# EUROPEAN PATENT SPECIFICATION

(11) **EP 2 202 299 B1**
(45) Date of publication and mention of the grant of the patent: **25.01.2012**
(21) Application number: 09180108.4
(22) Date of filing: 21.12.2009
(51) Int. Cl.: C12N 9/02, C12P 13/08

(54) **A method for producing L-lysine**
Verfahren zur Herstellung von L-Lysin
Procédé de production de L-Lysine

(30) Priority: 22.12.2008 JP 2008325952
(43) Date of publication of application: 30.06.2010
(73) Proprietor: Ajinomoto Co., Inc., Tokyo 104-8315 (JP)
(72) Inventor: Nagai, Yuri, Kanagawa 210-8681 (JP); Masumitsu, Yuri, Kanagawa 210-8681 (JP)
(74) Representative: HOFFMANN EITLE

(56) References cited:
- WO-A1-02/074944
- WO-A1-2006/071086
- WO-A2-02/20542
- US-A1- 2002 082 403
- RITTMANN DORIS ET AL: "Engineering of a glycerol utilization pathway for amino acid production by Corynebacterium glutamicum" APPLIED AND ENVIRONMENTAL MICROBIOLOGY, vol. 74, no. 20, October 2008 (2008-10), pages 6216-6222, XP002578554 ISSN: 0099-2240
- DATABASE EMBL [Online] 15 November 2003 'Escherichia coli strain TA234 glyceraldehyde phosphate dehydrogenase (gapA) gene, partial cds.' Retrieved from EBI, accession no. EMBL:AY301110 Database accession no. AY301110

### SEQUENCE LISTING

<110> Ajinomoto Co., Inc.
<120> A method for producing L-lysine
<130> OP-C9352
<150> JP2008-325952 <151> 2008-12-22
<160> 4
<170> PatentIn version 3.5
<210> 1
   <211> 996
   <212> DNA
   <213> Escherichia coli
<220>
   <221> CDS
   <222> (1).. (996)
<400> 1
   atg act atc aaa gta ggt atc aac ggt ttt ggc cgt atc ggt cgc att      48
   Met Thr Ile Lys Val Gly Ile Asn Gly Phe Gly Arg Ile Gly Arg Ile
   1               5                   10                  15
   gtt ttc cgt gct gct cag aaa cgt tct gac atc gag atc gtt gca atc      96
   Val Phe Arg Ala Ala Gln Lys Arg Ser Asp Ile Glu Ile Val Ala Ile
               20                  25                  30
   aac gac ctg tta gac gct gat tac atg gca tac atg ctg aaa tat gac     144
   Asn Asp Leu Leu Asp Ala Asp Tyr Met Ala Tyr Met Leu Lys Tyr Asp
           35                  40                  45
   tcc act cac ggc cgt ttc gac ggt acc gtt gaa gtg aaa gac ggt cat     192
   Ser Thr His Gly Arg Phe Asp Gly Thr Val Glu Val Lys Asp Gly His
       50                  55                  60
   ctg atc gtt aac ggt aaa aaa atc cgt gtt acc gct gaa cgt gat ccg     240
   Leu Ile Val Asn Gly Lys Lys Ile Arg Val Thr Ala Glu Arg Asp Pro
   65                  70                  75                  80
   gct aac ctg aaa tgg gac gaa gtt ggt gtt gac gtt gtc gct gaa gca     288
   Ala Asn Leu Lys Trp Asp Glu Val Gly Val Asp Val Val Ala Glu Ala
                   85                  90                  95
   act ggt ctg ttc ctg act gac gaa act gct cgt aaa cac atc acc gct     336
   Thr Gly Leu Phe Leu Thr Asp Glu Thr Ala Arg Lys His Ile Thr Ala
               100                 105                 110
   ggt gcg aag aaa gtg gtt atg act ggt ccg tct aaa gac aac act ccg      384
   Gly Ala Lys Lys Val Val Met Thr Gly Pro Ser Lys Asp Asn Thr Pro
           115                 120                 125
   atg ttc gtt aaa ggc gct aac ttc gac aaa tat gct ggc cag gac atc      432
   Met Phe Val Lys Gly Ala Asn Phe Asp Lys Tyr Ala Gly Gln Asp Ile
       130                 135                 140
   gtt tcc aac gct tcc tgc acc acc aac tgc ctg gct ccg ctg gct aaa      480
   Val Ser Asn Ala Ser Cys Thr Thr Asn Cys Leu Ala Pro Leu Ala Lys
   145                 150                 155                 160
   gtt atc aac gat aac ttc ggc atc atc gaa ggt ctg atg acc acc gtt      528
   Val Ile Asn Asp Asn Phe Gly Ile Ile Glu Gly Leu Met Thr Thr Val
                   165                 170                 175
   cac gct act acc gct act cag aaa acc gtt gat ggc ccg tct cac aaa      576
   His Ala Thr Thr Ala Thr Gln Lys Thr Val Asp Gly Pro Ser His Lys
               180                 185                 190
   gac tgg cgc ggc ggc cgc ggc gct tcc cag aac atc atc ccg tcc tct      624
   Asp Trp Arg Gly Gly Arg Gly Ala Ser Gln Asn Ile Ile Pro Ser Ser
           195                 200                 205
   acc ggt gct gct aaa gct gta ggt aaa gta ctg cca gaa ctg aat ggc      672
   Thr Gly Ala Ala Lys Ala Val Gly Lys Val Leu Pro Glu Leu Asn Gly
       210                 215                 220
   aaa ctg act ggt atg gcg ttc cgc gtt ccg acc ccg aac gta tct gta      720
   Lys Leu Thr Gly Met Ala Phe Arg Val Pro Thr Pro Asn Val Ser Val
   225                 230                 235                 240
   gtt gac ctg acc gtt cgt ctg gaa aaa gct gca act tac gag cag atc      768
   Val Asp Leu Thr Val Arg Leu Glu Lys Ala Ala Thr Tyr Glu Gln Ile
                   245                 250                 255
   aaa gct gcc gtt aaa gct gct gct gaa ggc gaa atg aaa ggc gtt ctg      816
   Lys Ala Ala Val Lys Ala Ala Ala Glu Gly Glu Met Lys Gly Val Leu
               260                 265                 270
   ggc tac acc gaa gat gac gta gta tct acc gat ttc aac ggc gaa gtt      864
   Gly Tyr Thr Glu Asp Asp Val Val Ser Thr Asp Phe Asn Gly Glu Val
           275                 280                 285
   tgc act tcc gtg ttc gat gct aaa gct ggt atc gct ctg aac gac aac      912
   Cys Thr Ser Val Phe Asp Ala Lys Ala Gly Ile Ala Leu Asn Asp Asn
       290                 295                 300
   ttc gtg aaa ctg gta tcc tgg tac gac aac gaa acc ggt tac tcc aac      960
   Phe Val Lys Leu Val Ser Trp Tyr Asp Asn Glu Thr Gly Tyr Ser Asn
   305                 310                 315                 320
   aaa gtt ctg gac ctg atc gct cac atc tcc aaa taa                      996
   Lys Val Leu Asp Leu Ile Ala His Ile Ser Lys
                   325                 330
<210> 2
   <211> 331
   <212> PRT
   <213> Escherichia coli
<400> 2
   Met Thr Ile Lys Val Gly Ile Asn Gly Phe Gly Arg Ile Gly Arg Ile
   1               5                   10                  15
   Val Phe Arg Ala Ala Gln Lys Arg Ser Asp Ile Glu Ile Val Ala Ile
               20                  25                  30
   Asn Asp Leu Leu Asp Ala Asp Tyr Met Ala Tyr Met Leu Lys Tyr Asp
           35                  40                  45
   Ser Thr His Gly Arg Phe Asp Gly Thr Val Glu Val Lys Asp Gly His
       50                  55                  60
   Leu Ile Val Asn Gly Lys Lys Ile Arg Val Thr Ala Glu Arg Asp Pro
   65                  70                  75                  80
   Ala Asn Leu Lys Trp Asp Glu Val Gly Val Asp Val Val Ala Glu Ala
                   85                  90                  95
   Thr Gly Leu Phe Leu Thr Asp Glu Thr Ala Arg Lys His Ile Thr Ala
               100                 105                 110
   Gly Ala Lys Lys Val Val Met Thr Gly Pro Ser Lys Asp Asn Thr Pro
           115                 120                 125
   Met Phe Val Lys Gly Ala Asn Phe Asp Lys Tyr Ala Gly Gln Asp Ile
       130                 135                 140
   Val Ser Asn Ala Ser Cys Thr Thr Asn Cys Leu Ala Pro Leu Ala Lys
   145                 150                 155                 160
   Val Ile Asn Asp Asn Phe Gly Ile Ile Glu Gly Leu Met Thr Thr Val
                   165                 170                 175
   His Ala Thr Thr Ala Thr Gln Lys Thr Val Asp Gly Pro Ser His Lys
               180                 185                 190
   Asp Trp Arg Gly Gly Arg Gly Ala Ser Gln Asn Ile Ile Pro Ser Ser
           195                 200                 205
   Thr Gly Ala Ala Lys Ala Val Gly Lys Val Leu Pro Glu Leu Asn Gly
       210                 215                 220
   Lys Leu Thr Gly Met Ala Phe Arg Val Pro Thr Pro Asn Val Ser Val
   225                 230                 235                 240
   Val Asp Leu Thr Val Arg Leu Glu Lys Ala Ala Thr Tyr Glu Gln Ile
                   245                 250                 255
   Lys Ala Ala Val Lys Ala Ala Ala Glu Gly Glu Met Lys Gly Val Leu
               260                 265                 270
   Gly Tyr Thr Glu Asp Asp Val Val Ser Thr Asp Phe Asn Gly Glu Val
           275                 280                 285
   Cys Thr Ser Val Phe Asp Ala Lys Ala Gly Ile Ala Leu Asn Asp Asn
       290                 295                 300
   Phe Val Lys Leu Val Ser Trp Tyr Asp Asn Glu Thr Gly Tyr Ser Asn
   305                 310                 315                 320
   Lys Val Leu Asp Leu Ile Ala His Ile Ser Lys
                   325                 330
<210> 3
   <211> 51
   <212> DNA
   <213> Artificial Sequence
<220>
   <223> primer
<400> 3
   ccatgattac gaattaggag gatatatgac tatcaaagta ggtatcaacg g 51
<210> 4
   <211> 41
   <212> DNA
   <213> Artificial Sequence
<220>
   <223> primer
<400> 4
   gatccccggg taccgttatt tggagatgtg agcgatcagg t 41

## Description

### TECHNICAL FIELD

The present invention relates to a method for producing L-lysine using a bacterium. L-Lysine is an industrially useful L-amino acid as an additive for animal feed, component of health food, amino acid infusion, and so forth.

### BACKGROUND ART

L-Lysine is industrially produced by fermentation using L-lysine-producing bacteria such as coryneform bacteria and *Escherichia* bacteria having an ability to produce L-lysine. As such L-lysine-producing bacteria, strains isolated from the nature, artificial mutant strains of such strains, recombinant strains in which L-lysine biosynthesis enzyme activity is enhanced by genetic recombination, and so forth are used in order to obtain improved productivity. Examples of method for producing L-lysine include, for example, the methods described in U.S. Patent Nos. 6,221,636 and 6,040,160, European Patent Laid-open Nos. 1158043 and 1063288

When amino acids are produced using a microorganism, sugars are generally used as a main component of carbon source, but glycerol can also be used as a carbon source like sugars (European Patent Laid-open Nos. 1715055 and 1715056).

Glyceraldehyde 3-phosphate dehydrogenase (henceforth also referred to as "GAPDH") is an enzyme of the glycolysis system, and it catalyzes a reaction for reversibly generating 1,3-diphosphoglycerate from glyceraldehyde-3-phosphate. In enterobacteria such as *Escherichia* bacteria and *Salmonella* bacteria, GAPDH is encoded by *gapA* genes, and although those *gapA* genes show polymorphism, nucleotide sequences of those genes and encoded amino acid sequences remain to show high homology (Proc. Natl. Acad. Sci. USA, 88, 6667-6671 (1991)).

As for use of the *gapA* gene, a technique of improving L-threonine producing ability by enhancing expression of a glycolysis system enzyme gene such as the *gapA* gene in L-threonine-producing bacteria (WO05/078113) is known. In this publication, glycerol is exemplified as a carbon source used for culture of those L-threonine-producing bacteria.

Further, there has been disclosed a method for producing an amino acid such as L-lysine using a coryneform bacterium in which expression of genes coding for various enzymes including GAPDH is enhanced (U.S. Patent Published Application No. 2002/0058277).

WO 2006/071086 describes Escherichia or Corynebacterium microorganisms comprising foreign NADP dependent glyceraldehyde 3-phosphate dehydrogenase gene and a method for producing L-lysine using the same.

WO 02/20542 describes an isolated polynucleotide coding for the gap2 gene of coryneform bacteria, and mentions a process for producing L-lysine comprising the use of coryneform bacteria overexpressing an endogenous *gap2* gene.

However, effect of enhancement of the GAPDH activity in L-lysine-producing *Escherichia coli* on L-lysine productivity is not known.

### SUMMARY OF THE INVENTION

An object of the present invention is to provide an *Escherichia coli* that can efficiently produce L-lysine using glycerol as a carbon source, and a method for efficiently producing L-lysine using such a bacterium, preferably such a method for efficiently producing L-lysine using glycerol as a carbon source.

The inventors of the present invention conducted various researches in order to achieve the aforementioned object. As a result, they found that if a bacterium was modified to increase the GAPDH activity, L-lysine productivity thereof was improved, especially when glycerol was used as a carbon source, and accomplished the present invention.

Thus, the present invention is as follows.
(1) A method for producingL-lysine, comprising culturing an *Escherichia coli* which has L-lysine-producing ability and has been modified to increase glyceraldehyde 3-phosphate dehydrogenase activity in a medium to produce and accumulate L-lysine in culture, and collecting L-lysine from the culture, wherein the glyceraldehyde 3-phosphate dehydrogenase activity is increased by increasing the expression of a *gapA* gene by increasing copy number of the gene or modifying an expression control sequence of the gene, and wherein the *gapA* gene is a DNA defined in the following (a) to (d):
   (a) a DNA comprising the nucleotide sequence of SEQ ID NO: 1,
   (b) a DNA hybridizable with a complementary sequence of the nucleotide sequence of SEQ ID NO: 1 under stringent conditions comprising washing at salt concentrations and temperature of 0.1 X SSC, 0.1% SDS at 60°C, and encoding a protein having the glyceraldehyde 3-phosphate dehydrogenase activity,
   (c) a DNA coding for a protein having the amino acid sequence of SEQ ID NO: 2, or
   (d) a DNA coding for a protein having the amino acid sequence of SEQ ID NO: 2 which includes substitutions, deletions, insertions, additions or inversions of one to 20 amino acid residues, and having glyceraldehyde 3-phosphate dehydrogenase activity.
2. The method according to item 1, wherein the medium contains glycerol as a carbon source.

### DETAILED DESCRIPTION OF THE PREFERRED EMBODIMENTS

### <1> Bacterium for use in the method of present invention

The bacterium for use in a method of the present invention is an *Escherichia coli* having an L-lysine-producing ability and modified to incrase the GAPDH activity is increased.

In the present invention, the L-lysine-producing ability refers to an ability of the bacterium for use in a method of the present invention to produce and accumulate L-lysine in a medium or cells thereof to such a degree that L-lysine can be collected from the medium or cells when the bacterium is cultured in the medium. The bacterium having an L-lysine producing ability may be a bacterium originally having the L-lysine producing ability, or may be a bacterium obtained by modifying a bacterium such as those mentioned below using a mutation technique or a recombinant DNA technique so that the bacterium has the L-lysine producing ability.

Further, in the present invention, L-lysine includes not only L-lysine in the free form, but also salts thereof such as L-lysine hydrochloride, L-lysine sulfate and L-lysine carbonate.

In the present invention, "increase of expression of a gene" means increase of transcription amount and/or translation amount of the gene.

Methods for imparting L-lysine-producing ability to a bacterium and *Escherichia coli* imparted with an L-lysine-producing ability, which can be used for the present invention, will be exemplified below. However, so long as an *Escherichia coli* having an L-lysine-producing ability is chosen, *Escherichia coli* is not limited to these.

The parent strain of *Escherichia coli* used for obtaining the *Escherichia coli* for use in a method of the present invention is not particularly limited. However, specific examples include, for example, those described in the work of Neidhardt et al. (Backmann B.J., 1996, Derivations and Genotypes of some mutant derivatives of Escherichia coli K-12, p.2460-2488, Table 1, In F.D. Neidhardt (ed.), Escherichia coli and Salmonella Cellular and Molecular Biology/Second Edition, American Society for Microbiology Press, Washington, D.C.) can be used. Specific examples include *Escherichia coli* W3110 (ATCC 27325), *Escherichia coli* MG1655 (ATCC 47076) and so forth derived from the prototype wild strain, K12 strain.

These strains are available from, for example, American Type Culture Collection (Address: P.O. Box 1549, Manassas, VA 20108, United States of America). That is, registration numbers are given to the strains, respectively, and the strains can be ordered by using these registration numbers (refer to http://www.atcc.org/). The registration numbers of the strains are listed in the catalogue of the American Type Culture Collection.

Methods for imparting L-lysine-producing ability to *Escherichia coli* and methods for enhancing L-lysine-producing ability of *Escherichia coli* will be described below.

To impart L-lysine-producing ability, methods conventionally employed in the breeding of coryneform bacteria or *Escherichia* bacteria (see "Amino Acid Fermentation", Gakkai Shuppan Center (Ltd.), 1st Edition, published on May 30, 1986, pp.77-100) can be used. Such methods include acquiring auxotrophic mutant strains, analogue-resistant strains, or metabolic regulation mutant strains, constructing recombinant strains in which expression of an L-lysine biosynthesis enzyme is increased, and so forth. In the breeding of L-lysine-producing bacteria, one or more of the properties including auxotrophy, analogue resistance, metabolic regulation mutation and so forth may be imparted. Similarly, expression of one or more L-lysine biosynthesis enzymes can be enhanced. Furthermore, the impartation of the properties such as auxotrophy, analogue resistance and metabolic regulation mutation may be combined with the enhancement of the biosynthesis enzymes.

Auxotrophic mutant strains, L-lysine analogue-resistant strains, or metabolic regulation mutant strains having an L-lysine-producing ability can be obtained by subjecting a parent or wild-type strain to a conventional mutatagenesis treatment, such as exposure to X-rays or UV irradiation, or treatment with a mutagen such as N-methyl-N'-nitro-N-nitrosoguanidine or ethyl methanesulfonate (EMS), and then selecting those which exhibit autotrophy, analogue resistance, or metabolic regulation mutation and which also have the L-lysine-producing ability from the obtained mutant strains.

L-Lysine-producing bacteria and methods for constructing them are exemplified below.

Examples of strains having L-lysine-producing ability include, for example, L-lysine analogue-resistant strains and metabolic regulation mutant strains. Examples of L-lysine analogue include, but are not limited to, oxalysine, lysine hydroxamate, S-(2-aminoethyl)-L-cysteine (AEC), γ-methyllysine, α-chlorocaprolactam and so forth. Mutant strains having resistance to these lysine analogues can be obtained by subjecting *Escherichia coli* to a conventional artificial mutagenesis treatment. Specific examples of L-lysine-producing bacteria include *Escherichia coli* AJ11442 (FERM BP-1543, NRRL B-12185, see Japanese Patent Laid-open (Kokai) No. 56-18596 and U.S. Patent No. 4,346,170), *Escherichia coli* VL611 strain (Japanese Patent Laid-open No. 2000-189180), and so forth. As an L-lysine-producing *Escherichia coli,* the WC196 strain may also be used (see International Patent Publication WO96/17930). The WC196 strain was bred from the W3110 strain, which was derived from *Escherichia coli* K-12, by replacing the wild type *lysC* gene on the chromosome of the W3110 strain with a mutant *lysC* gene encoding a mutant aspartokinase III in which threonine at position 352 had been replaced with isoleucine, resulting in desensitization of the feedback inhibition thereof by L-lysine (U.S. Patent No. 5,661,012), and conferring AEC resistance to the resulting strain (U.S. Patent No. 5,827,698). The WC196 strain was designated *Escherichia coli* AJ13069, deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6, 1994, and assigned an accession number of FERM P-14690. Then, it was converted to an international deposit under the provisions of the Budapest Treaty on September 29, 1995, and assigned an accession number of FERM BP-5252 (U.S. Patent No. 5,827,698).

L-Lysine-producing bacteria can also be constructed by enhancing activity of an L-lysine biosynthetic enzyme. Activity of such an enzyme can be enhanced by increasing copy number of a gene encoding the enzyme in cells or by modifying an expression control sequence of the gene encoding the enzyme. Increase of copy number of the gene coding for an enzyme of L-lysine biosynthesis in cells and modification of an expression control sequence can be attained in the same manner as those for the *gapA* gene described below.

Examples of genes encoding L-lysine biosynthetic enzymes include genes encoding enzymes of the diaminopimelate pathway such as dihydrodipicolinate synthase gene (*dapA*), aspartokinase gene (*lysC*), dihydrodipicolinate reductase gene (*dapB*), diaminopimelate decarboxylase gene (*lysA*), diaminopimelate dehydrogenase gene (ddh) (WO96/40934 for all the foregoing genes), phosphoenolpyrvate carboxylase gene (*ppc*) (Japanese Patent Laid-open No. 60-87788), aspartate aminotransferase gene (*aspC*) (Japanese Patent Publication (Kokoku) No. 6-102028), diaminopimelate epimerase gene (*dapF*) (Japanese Patent Laid-open No. 2003-135066), and aspartate semialdehyde dehydrogenease gene (*asd*) (WO00/61723), and genes encoding enzymes of the aminoadipic acid pathway such as homoaconitate hydratase gene (Japanese Patent Laid-open No. 2000-157276). In addition, the parent strain may show an increased level of expression of the gene involved in energy efficiency (*cyo*) (EP 1170376 A), the gene coding for nicotinamide nucleotide transhydrogenase (*pntAB*) (U.S. Patent No. 5,830,716), the *ybjE* gene encoding a protein having L-lysine excretion activity (WO2005/073390), the gene encoding glutamate dehydrogenase (*gdhA*) (Gene, 23:199-209 (1983)), or an arbitrary combination of these. Abbreviations for the genes are shown in the parentheses.

It is known that the wild-type dihydrodipicolinate synthase derived from *Escherichia coli* is subject to feedback inhibition by L-lysine, and it is known that the wild-type aspartokinase derived from *Escherichia coli* is not subject to suppression and feedback inhibition by L-lysine. Therefore, when the *dapA* gene and *lysC* gene are used, these genes are preferably genes coding for mutant enzymes desensitized to the feedback inhibition by L-lysine.

Examples of DNA encoding a mutant dihydrodipicolinate synthetase desensitized to feedback inhibition by L-lysine include a DNA encoding such a protein having an amino acid sequence in which the histidine residue at the position 118 is replaced by tyrosine residue. Examples of DNA encoding a mutant aspartokinase desensitized to feedback inhibition by L-lysine include a DNA encoding an AKIII having an amino acid sequence in which the threonine residue at the position 352, the glycine residue at the position 323, and the methionine residue at the position 318 are replaced by isoleucine, asparagine and isoleucine residues, respectively (for these mutants, see U.S. Patent Nos. 5,661,012 and 6,040,160). Such mutant DNAs can be obtained by site-specific mutagenesis using PCR or the like.

Wide host-range plasmids RSFD80, pCAB1, and pCABD2 are known as plasmids containing a mutant *dapA* gene encoding a mutant dihydrodipicolinate synthase and a mutant *lysC* gene encoding a mutant aspartokinase (U.S. Patent No. 6,040,160). *Escherichia coli* JM109 strain transformed with RSFD80 was named AJ12396 (U.S. Patent No. 6,040,160), and the strain was deposited at the National Institute of Bioscience and Human-Technology, Agency of Industrial Science and Technology, Ministry of International Trade and Industry (currently National Institute of Advanced Industrial Science and Technology, International Patent Organism Depositary) on October 28, 1993 and assigned an accession number of FERM P-13936, and the deposit was then converted to an international deposit under the provisions of Budapest Treaty on November 1, 1994 and assigned an accession number of FERM BP-4859. RSFD80 can be obtained from the AJ12396 strain by a conventional method.

Furthermore, L-lysine-producing bacteria may have reduced activity of an enzyme that catalyzes a reaction branching off from the L-lysine biosynthesis pathway and producing a compound other than L-lysine or may be deficient in such an activity, or they may have reduced activity of an enzyme that negatively acts on L-lysine synthesis or accumulation, or may be deficient in such an activity. Examples of such enzymes involved in the L-lysine production include homoserine dehydrogenase, lysine decarboxylase (*cadA, ldcC*), malic enzyme, and so forth, and strains in which activities of these enzymes are decreased or deleted are disclosed in WO95/23864, WO96/17930, WO2005/010175, and so forth.

It is preferred that expressions of both the *cadA* and *ldcC* genes encoding lysine decarboxylase are decreased in order to decrease or delete the lysine decarboxylase activity.

In order to reduce or eliminate activities of these enzymes, a mutation may be introduced into genes of the enzymes on a genome by a usual mutagenesis method or gene recombination technique so that intracellular activities of the enzymes are reduced or eliminated. Such introduction of a mutation can be achieved by, for example, using genetic recombination to eliminate the genes coding for the enzymes on the genome or to modify an expression control sequence such as a promoter or the Shine-Dalgarno (SD) sequence. It can also be achieved by introducing a mutation for amino acid substitution (missense mutation), a stop codon (nonsense mutation), or a frame shift mutation for adding or deleting one or two nucleotides into regions coding for the enzymes on the genome, or partially or totally deleting the genes (J. Biol. Chem., 272:8611-8617 (1997)). The enzymatic activities can also be decreased or eliminated by constructing a mutant gene of which coding region of enzyme gene is entirely or partially deleted, and substituting it for a normal gene on a genome by homologous recombination or the like, or by introducing a transposon or IS factor into the enzyme genes.

For example, in order to introduce a mutation that decreases or eliminates the activities of the above-mentioned enzymes by genetic recombination, the following methods are used. A mutant gene is prepared by modifying a partial sequence of an target gene so that it does not encode an enzyme that can function normally, and then *Escherichia coli* can be transformed with a DNA containing the mutant gene to cause recombination of a corresponding gene on the genome with the mutant gene to substitute the mutant gene for the target gene on the genome. Examples of such gene substitution using homologous recombination include methods of using a linear DNA such as the method called Red-driven integration (Datsenko, K.A, and Wanner, B.L., Proc. Natl. Acad. Sci. USA, 97:6640-6645 (2000)), and the method utilizing the Red driven integration in combination with an excisive system derived from λ phage (Cho, E.H., Gumport, R.I., Gardner, J.F., J. Bacteriol., 184:5200-5203 (2002)), a method of using a plasmid containing a temperature sensitive replication origin (U.S. Patent No. 6,303,383, Japanese Patent Laid-open No. 05-007491), and so forth. Further, such site-specific mutagenesis based on gene substitution using homologous recombination can also be performed by using a plasmid which is not able to replicate in a host.

Preferred examples of L-lysine-producing bacteria include *Escherichia coli* WC196ΔcadAΔldc/pCABD2 (WO2006/078039). The WC196ΔcadAΔldc/pCABD2 strain was constructed by introducing the plasmid pCABD2 containing lysine biosynthesis genes (U.S. Patent No. 6,040,160) into the WC196 strain having disrupted *cadA* and *ldcC* genes, which encode lysine decarboxylase. The WC196 strain was bred from the W3110 strain, which was derived from *Escherichia coli* K-12, by replacing the wild type *lysC* gene on the chromosome of the W3110 strain with a mutant *lysC* gene encoding a mutant aspartokinase III in which threonine at position 352 had been replaced with isoleucine, resulting in desensitization of the feedback inhibition thereof by L-lysine (U.S. Patent No. 5,661,012), and conferring AEC resistance to the resulting strain (U.S. Patent No. 5,827,698). The WC196 strain was designated *Escherichia coli* AJ13069, deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6, 1994, and assigned an accession number of FERM P-14690. Then, it was converted to an international deposit under the provisions of the Budapest Treaty on September 29, 1995, and assigned an accession number of FERM BP-5252 (U.S. Patent No. 5,827,698). The WC196ΔcadAΔldc strain itself is also a preferred L-lysine-producing bacterium. The WC196ΔcadAΔldc was designated AJ110692, and deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on October 7, 2008 as an international deposit and assigned an accession number of FERM BP-11027.

The plasmid pCABD2 contains a mutant *dapA* gene derived from *Escherichia coli* which encodes dihydrodipicolinate synthase (DDPS) have a mutation for desensitization to the feedback inhibition by L-lysine, a mutant *lysC* gene derived from *Escherichia coli* which encodes aspartokinase III having a mutation for desensitization to the feedback inhibition by L-lysine, the *dapB* gene derived from *Escherichia coli* which encodes dihydrodipicolinate reductase, and the *ddh* gene derived from *Brevibacterium lactofermentum* (Corynebacterium glutamicum) which encodes diaminopimelate dehydrogenase.

In the L-lysine-producing bacterium used for the present invention, besides genes coding for L-lysine biosynthesis enzymes, genes involved in the glycerol metabolism, i.e., genes coding for enzymes of the glycolysis system downstream from 1,3-diphosphoglycerate, may be modified.

As for the gene involved in the glycerol metabolism, in order to enhance glycerol assimilability, expression of the *glpR* gene (EP 1715056) may be attenuated, or expression of glycerol metabolism genes such as *glpA, glpB, glpC, glpD, glpE, glpF, glpG, glpK, glpQ, glpT, glpX, tpiA, gldA, dhaK, dhaL, dhaM, dhaR, fsa* and *talC* genes (EP 1715055 A) may be enhanced.

In particular, in order to enhance glycerol assimilating property, it is preferred that expression of the glycerol dehydrogenase gene (*gldA*), dihydroxyacetone kinase gene (*dhaKLM, dak*) and fructose-6-phosphate aldolase gene (*fsaB*) is enhanced (WO2008/102861).

Further, as for glycerol kinase (*glpK*), it is preferable to use a desensitized type *glpK* gene for desensitization to the feedback inhibition by fructose-1,6-phosphate (WO2008/081959, WO2008/107277)

Examples of the gene coding for an enzyme of the glycolysis system downstream from 1,3-diphosphoglycerate include the pyruvate kinase gene (*pykF*, WO03/008609), transaldolase gene (*talB*, WO03/008611), fumarase gene (*fum,* WO01/02545), and phosphoenolpyruvate synthase gene (*pps,* European Patent Laid-open No. 877090).

The bacterium for use in a method of the present invention can be obtained by modifying a bacterium having an L-lysine-producing ability such as those described above so that GAPDH activity is increased. The bacterium for use in a method of the present invention can also be obtained by imparting the L-lysine-producing ability to a bacterium which has been modified to increase GAPDH activity.

Enhancement of the GAPDH activity can be attained by such modification that expression of the gene coding for GAPDH increases, and this increase of expression may be enhancement of expression of an endogenous gene attained by modification of an expression control region such as modification of a promoter, or may be enhancement of expression of an exogenous gene attained by incorporation of a plasmid containing the gene or the like. Furthermore, these may be used in combination.

The GAPDH activity referred to herein means an activity for catalyzing a reaction of generating 1,3-diphosphoglycerate from glyceraldehyde-3-phosphate, and the state of "being modified to increase GAPDH activity " corresponds to a state that number of the GAPDH molecules per cell increases or the GAPDH activity per molecule improves as compared to an unmodified strain such as a wild-type or parent strain. The modification is preferably performed so that the GAPDH activity per cell increases to 150% or more, more preferably 200% or more, still more desirably 300% or more, of the activity of an unmodified strain. Examples of unmodified strain such as a wild-type strain of *Escherichia coli* serving as a reference for the above comparison include, for example, the *Escherichia coli* MG1655 strain (ATCC 47076) and W3110 strain (ATCC 27325) as wild-type strains of *Escherichia coli, Pantoea ananatis* AJ13335 strain (FERM BP-6614, European Patent Laid-open No. 0952221), and so forth. The GAPDH activity can be measured by, for example, the method described in The Journal of Biological Chemistry, 239, 2140 (1963), a method using coupling with phosphoglycerate kinase (PGK) as shown by the following reaction formula, or the like.

### 3-Phosphoglycerate <=> glycerate-1,3-biphoshate <=> glyceraldehyde-3-phosphate

Enhancement of the GAPDH activity can be attained by such modification that expression of a gene coding for GAPDH increases. As a gene coding for GAPDH of *Escherichia coli,* the *gapA* gene is known. The nucleotide sequence of the gene and the encoded amino acid sequence are shown in SEQ ID NOS: 1 and 2, respectively.

Increase of expression of the gene coding for GAPDH (*gapA* gene) compared with that of an unmodified strain, for example, a parent or wild-type strain, can be confirmed by comparing amount of mRNA of the gene with that of the wild-type or the unmodified strain. Examples of the method for confirming increase of the expression include Northern hybridization and reverse-transcriptase PCR (RT-PCR, Sambrook, J., and Russell, D.W., Molecular Cloning A Laboratory Manual/Third Edition, New York: Cold Spring Harbor Laboratory Press (2001)). Expression of the gene may be increased to any level so long as it increases as compared to that of an unmodified strain, and for example, it is desirably increased not less than 1.5 times, more preferably not less than 2 times, further preferably not less than 3 times, as compared to that of, for example, an unmodified strain. Moreover, increase of the expression can also be confirmed on the basis of increase in amount of the GAPDH protein compared with that in an unmodified strain, and it can be detected by, for example, Western blotting using an antibody (Sambrook, J., and Russell, D.W., Molecular Cloning A Laboratory Manual/Third Edition, New York: Cold Spring Harbor Laboratory Press (2001)).

The *gapA* gene of the present invention means the *gapA* gene of an *Escherichia* bacterium or a homologue thereof. As the *gapA* gene of *Escherichia coli,* a gene coding for a protein having the amino acid sequence of SEQ ID NO: 2 (SEQ ID NO: 1, GenBank accession NC_000913.2, GI:49175990, nucleotide numbers 1860795-1861790) can be exemplified.

A homologue of the *gapA* gene means a gene derived from another microorganism, showing high homology to the *gapA* gene of an *Escherichia* bacterium and coding for a protein showing the GAPDH activity, when it is introduced into a host.

Examples of the *gapA* homologue include, for example, the *gapA* genes from *Salmonella* bacteria and the like registered at GenBank. Further, the *gapA* gene may be a gene cloned from the genus *Saccharomyces* on the basis of homology to the genes exemplified above. Any gene showing high homology to the *gapA* of an *Escherichia* bacterium may be used, even though a different gene name may be given to the gene. The *gapA* gene homologues include, for example, genes that can be cloned by using the synthetic oligonucleotides of SEQ ID NOS: 3 and 4.

Further, as a *gapA* gene homologue, a gene showing high homology can be obtained from a known database on the basis of the aforementioned sequence information. Homology of amino acid sequences and nucleotide sequences can be determined by using, for example, the algorithm BLAST of Karlin and Altschul (Pro. Natl. Acad. Sci. USA, 90, 5873 (1993)) or FASTA (Methods Enzymol., 183, 63 (1990)). Programs called BLASTN and BLASTX have been developed on the basis of this algorithm BLAST (refer to www.ncbi.nlm.nih.gov). In this specification, the term "homology" may also be used to refer to "identity".

Further, the *gapA* gene used for the present invention is not limited to a wild-type gene, and it may be a mutant or artificially modified gene coding for a protein having an amino acid sequence of SEQ ID NO: 2 including substitutions, deletions, insertions, additions or the like of one or several amino acid residues at one or more positions so long as the function of the encoded protein is not degraded.

Although number meant by the term "one or several" used herein may differ depending on positions in the three-dimensional structure of the protein or types of amino acid residues, specifically, it may be 1 to 20, preferably 1 to 10, more preferably 1 to 5. The aforementioned substitutions, deletions, insertions or additions of one or several amino acid residues is a conservative mutation which maintains the GAPDH activity. The conservative mutation is typically a conservative substitution. The conservative substitution is a substitution wherein substitution takes place mutually among Phe, Trp and Tyr, if the substitution site is an aromatic amino acid; among Leu, Ile and Val, if the substitution site is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having hydroxyl group. Specific examples of substitution considered conservative substitution include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Gly, Asn, Gln, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val. The mutation for such substitution, deletion, insertion, addition, inversion or the like of amino acid residues as described above also includes a naturally occurring mutation based on individual difference, difference in species of microorganisms having the *gapA* gene (mutant or variant) and so forth. A gene having such a mutation can be obtained by modifying the nucleotide sequence of SEQ ID NO: 1 by, for example, site-specific mutagenesis, so that substitution, deletion, insertion or addition of an amino acid residue or residues is included in the encoded protein at a specific site.

Further, as the *gapA* gene, a gene coding for a protein showing a homology of 90% or more, preferably 95% or more, more preferably 97% or more, particularly preferably 98% or more, further preferably 99% or more, to the entire amino acid sequence of SEQ ID NO: 2 may be used.

Furthermore, codons of the *gapA* gene may be replaced with those which are easily used by a host into which the *gapA* gene is introduced. Moreover, so long as the GAPDH activity is maintained, the *gapA* gene may be a gene coding for a protein of which N- or C-terminus sequence is elongated or deleted. The length of amino acid sequence to be elongated or deleted may be 50 or less, preferably 20 or less, more preferably 10 or less, particularly preferably 5 or less, in terms of number of amino acid residues. More specifically, the *gapA* gene may be a gene coding for a protein having the amino acid sequence of SEQ ID NO: 2 with elongation or deletion of 5 to 50 amino acid residues on the N-terminal side or 5 to 50 amino acid residues on the C-terminal side.

Moreover, a modified *gapA* gene can also be obtained by the conventionally known mutation treatment described below. Examples of the mutation treatment include a method of treating the *gapA* gene with hydroxylamine or the like in vitro, and a method of treating a microorganism, for example, *Escherichia* bacteria, containing the gene with ultraviolet ray irradiation or a mutagen used in a conventional mutagenesis such as N-methyl-N'-nitro-N-nitrosoguanidine (NTG) or ethyl methanesulfonate (EMS).

Moreover, the *gapA* gene may also be a DNA hybridizable with a sequence complementary to the nucleotide sequence of SEQ ID NO: 1 or a probe that can be prepared from such sequences under stringent conditions.

The term "stringent conditions" referred to herein means conditions under which specific hybrids are formed, but non-specific hybrids are not formed. Although it is difficult to definitely define with numerals, examples include, for example, conditions under which DNAs showing high homology to each other, for example, DNAs showing a homology of not less than 80%, preferably not less than 90%, more preferably not less than 95%, particularly preferably not less than 97%, hybridize with each other, and DNAs having homology lower than the above level do not hybridize with each other, and conditions of washing in ordinary Southern hybridization, i.e., conditions of washing once, preferably twice or three times, at salt concentrations and temperature of 1 x SSC, 0.1% SDS at 60°C, preferably 0.1 x SSC, 0.1% SDS at 60°C, more preferably 0.1 x SSC, 0.1% SDS at 68°C.

As the probe, a part of the complementary sequence of the sequence of SEQ ID NO: 1 may also be used. Such a probe can be produced by PCR using oligonucleotides prepared on the basis of the sequence of SEQ ID NO: 1 as primers and a DNA fragment containing the nucleotide sequence as a template. When a DNA fragment having a length of about 300 bp is used as the probe, the washing condition after hybridization can be exemplified by 2 x SSC, 0.1% SDS at 50°C.

The modification for enhancing expression of the *gapA* gene can be attained by increasing copy number of the *gapA* gene in cells utilizing, for example, gene recombination techniques. For example, the copy number of the gene may be increased by ligating a DNA fragment containing the *gapA* gene to a vector which functions in a host bacterium, preferably a multi copy vector, to prepare a recombinant DNA, and introducing it into a bacterium to transform the bacterium.

When the *gapA* gene of *Escherichia coli* is used as the *gapA* gene, it can be obtained by PCR (polymerase chain reaction, refer to White, T.J. et al., Trends Genet., 5, 185 (1989)) using primers prepared on the basis of the nucleotide sequence of SEQ ID NO: 1, for example, the primers shown in SEQ ID NOS: 3 and 4, and a genomic DNA of *Escherichia coli* as a template. The *gapA* genes derived from other bacteria belonging to the family *Enterobacteriaceae* can also be obtained from a genomic DNA or a genomic DNA library of each microorganism by PCR using, as primers, oligonucleotides prepared based on sequence information of the *gapA* gene known for the bacterium or a bacterium of another species, or hybridization using an oligonucleotide prepared based on such sequence information as described above as a probe. A genomic DNA can be prepared from a microorganism that serves as a DNA donor by the method of Saito and Miura (Saito H. and Miura K., 1963, Biochem. Biophys. Acta, 72, 619; Experiment Manual for Biotechnology, edited by The Society for Biotechnology, Japan, pp.97-98, Baifukan Co., Ltd., 1992) or the like.

Then, the *gapA* gene amplified by PCR is ligated to a vector DNA which can function in host bacterial cells to prepare a recombinant DNA. Examples of the vector which can function in host bacterial cells include vectors autonomously replicable in the host bacterial cells. Examples of a vector which is autonomously replicable in *Escherichia coli* cells include pUC19, pUC18, pHSG299, pHSG399, pHSG398, pACYC184, (pHSG and pACYC series vectors are available from Takara Bio Inc.), RSF1010, pBR322, pMW219 (pMW219 is available form Nippon Gene Co., Ltd.), pSTV29 (available form Takara Bio Inc.), and so forth.

In order to introduce a recombinant DNA prepared as described above into a bacterium, any known transformation method reported to date can be employed. For example, there are a method of treating recipient cells with calcium chloride so as to increase permeability for DNA, which has been reported for *Escherichia coli* K-12 (Mandel, M. and Higa, A., J. Mol. Biol., 53, 159 (1970)), and a method of using competent cells prepared from growing cells and introducing DNA into them, which has been reported for *Bacillus subtilis* (Duncan, C.H., Wilson, G.A. and Young, F.E., Gene, 1, 153 (1977)). Also applicable are a method of making DNA recipient cells into protoplasts or spheroplasts, which easily take up a recombinant DNA, and introducing a recombinant DNA into them, which is known for *Bacillus subtilis,* actinomycetes, and yeasts (Chang, S. and Choen, S.N., Molec. Gen. Genet., 168, 111 (1979); Bibb, M.J., Ward, J.M. and Hopwood, O.A., Nature, 274, 398 (1978); Hinnen, A., Hicks, J.B. and Fink, G.R., Proc. Natl. Acad. Sci. USA, 75, 1929 (1978)), and electroporation (Canadian Journal of Microbiology, 43, 197 (1997)).

Copy number of the *gapA* can also be increased by integrating multiple copies of such a *gapA* gene as described above into a genomic DNA of a bacterium. In order to integrate multiple copies of the *gapA* gene into a genomic DNA of a bacterium, homologous recombination is performed by targeting a sequence which exists in multiple copies on the genomic DNA. As the sequence present on a genomic DNA in a multiple copy number, a repetitive DNA or inverted repeat present at the end of a transposable element can be used. The genes may be ligated aside the *gapA* gene existing in the genome in tandem, or they may also be introduced into an unnecessary gene so that the genes exist in a multiple number. Such gene transfer can be attained by using a temperature sensitive vector or an integration vector.

Alternatively, as disclosed in Japanese Patent Laid-open No. 2-109985, it is also possible to incorporate the *gapA* gene into a transposon, and allow it to be transferred to introduce multiple copies of the gene into a genomic DNA. Whether the gene has been transferred into a genome can be confirmed by performing Southern hybridization using a part of the *gapA* gene as a probe.

Furthermore, besides by increase of copy number of the gene described above, expression of the *gapA* gene may also be enhanced according to the method described in WO00/18935 by replacing an expression control sequence such as a promoter of the *gapA* gene on a genomic DNA or a plasmid with a stronger promoter, modifying the sequences of the -35 and -10 regions so that the sequences become consensus sequences, amplifying a regulator that increases expression of the *gapA* gene, or deleting or attenuating a regulator that decreases expression of the *gapA* gene. For example, the *lac* promoter, *trp* promoter, *trc* promoter, *tac* promoter, *araBA* promoter, lambda phage PR promoter and PL promoter, tet promoter, T7 promoter, ϕ10 promoter, and so forth are known as strong promoters. A promoter or SD sequence of the *gapA* gene can also be modified so as to become stronger by introducing a nucleotide substitution or the like. Examples of method for evaluating strength of a promoter and strong promoters are described in Goldstein et al. (Prokaryotic promoters in biotechnology, Biotechnol. Annu. Rev., 1, 105-128 (1995)) and so forth. In addition, it is known that substitution of several nucleotides in a spacer between the ribosome binding site (RBS) and translation initiation codon, especially a sequence immediately upstream from the initiation codon, greatly affect mRNA translation efficiency, and therefore this sequence may be modified. Expression control regions of the *gapA* gene may be identified by using a promoter probe vector or gene analysis software such as GENETYX.
By such substitution or modification of promoter as described above, expression of the *gapA* gene is enhanced. Substitution of an expression control sequence can also be attained by, for example, a method using a temperature sensitive plasmid or Red-driven integration (WO2005/010175).

### <2> Method for producing L-lysine

The method for producing L-lysine of the present invention is characterized by culturing the bacterium as above in a medium to produce and accumulate L-lysine in culture, and collecting L-lysine from the culture.

As the medium to be used, media conventionally used for the production of L-amino acids by fermentation using microorganisms can be used. That is, usual media containing a carbon source, a nitrogen source, inorganic ions, and optionally other organic components as required can be used. As the carbon source, saccharides such as glucose, sucrose, lactose, galactose, fructose, and hydrolysates of starches; alcohols such as glycerol and sorbitol; and organic acids such as fumaric acid, citric acid and succinic acid can be used. In addition, as for a strain not having sucrose-assimilating ability, if a sucrose assimilation gene is introduced into such a strain, it becomes possible to use sucrose as a carbon source (U.S. Patent No. 5,175,107).

In the present invention, it is preferable to use glycerol as the carbon source. Glycerol may be used at any concentration so long as a concentration suitable for production of L-lysine is chosen. When glycerol is used as a sole carbon source in the medium, it is preferably contained in the medium in an amount of about 0.1 to 50 w/v %, more preferably about 0.5 to 40 w/v %, particularly preferably about 1 to 30% w/v %. Glycerol can also be used in combination with other carbon sources such as glucose, fructose, sucrose, blackstrap molasses and starch hydrolysate. In this case, although glycerol and other carbon sources may be mixed at an arbitrary ratio, it is desirable that the ratio of glycerol in the carbon source is 10% by weight or more, more preferably 50% by weight or more, still more preferably 70% by weight or more. Preferred as the other carbon sources are saccharides such as glucose, fructose, sucrose, lactose, galactose, blackstrap molasses, starch hydrolysate and a sugar solution obtained by hydrolysis of biomass, alcohols such as ethanol, and organic acids such as fumaric acid, citric acid and succinic acid. Among these, glucose is preferred.

Although initial concentration of glycerol at the time of starting the culture is preferably as described above, glycerol may be supplemented with consumption of glycerol during the culture.

Glycerol to be used may be pure glycerol or crude glycerol. Crude glycerol refers to industrially produced glycerol containing impurities. Crude glycerol is industrially produced by contacting fats and oils with water under a high temperature and high pressure to hydrolyze them, or by the esterification reaction for biodiesel fuel production. Biodiesel fuel refers to aliphatic acid methyl esters produced from fats and oils and methanol by a transesterification reaction, and crude glycerol is produced as a by-product of this reaction (refer to Fukuda, H., Kondo, A., and Noda, H., 2001, J. Biosci. Bioeng., 92, 405-416). In the biodiesel fuel production process, the alkaline catalyst method is used for the transesterification in many cases, and acids are added for neutralization. Therefore, crude glycerol having a purity of about 70 to 95% by weight and containing water and impurities is produced. Crude glycerol produced in the biodiesel fuel production contains, in addition to water, residual methanol, salts of alkali such as NaOH as a catalyst and acid such as K₂SO₄ used for neutralizing the alkali as impurities. Although it depends on manufacturers and production methods, content of such salts and methanol reaches several percents. Crude glycerol preferably contains ions originating in the alkali and the acid used for the neutralization of the alkali such as sodium ions, potassium ions, chloride ions and sulfate ions in an amount of 2 to 7%, preferably 3 to 6%, more preferably 4 to 5.8%, based on the weight of the crude glycerol. Although methanol may not be contained as impurity, it is preferably contained desirably in an amount of 0.01% or less.

The crude glycerol may further contain trace amounts of metals, organic acids, phosphorus, aliphatic acids, and so forth. Examples of the organic acids contained include formic acid, acetic acid, and so forth, and although they may not be contained as impurities, they are preferably contained desirably in an amount of 0.01% or less. As the trace amounts of metals contained in the crude glycerol, trace metals required for growth of microorganisms are preferred, and examples include, for example, magnesium, iron, calcium, manganese, copper, zinc, and so forth. Magnesium, iron and calcium are preferably contained in an amount of 0.00001 to 0.1%, preferably 0.0005 to 0.1%, more preferably 0.004 to 0.05%, still more preferably 0.007 to 0.01%, in terms of the total amount based on the weight of the crude glycerol. Manganese, copper and zinc are preferably contained in an amount of 0.000005 to 0.01%, more preferably 0.000007 to 0.005%, still more preferably 0.00001 to 0.001%, in terms of the total amount(EP2003209).

The purity of the crude glycerol as glycerol may be 10% or higher, preferably 50% or higher, more preferably 70% or higher, particularly preferably 80% or higher. So long as the content of impurities is within the aforementioned range, the purity of the glycerol may be 90% or higher.

When crude glycerol is used, crude glycerol may be added to the medium according to the purity of the glycerol so that glycerol concentration is a concentration within the aforementioned range. Moreover, both glycerol and crude glycerol may be added to the medium.

As the nitrogen source, inorganic ammonium salts such as ammonium sulfate, ammonium chloride, and ammonium phosphate, organic nitrogen such as soybean hydrolysate, ammonia gas, aqueous ammonia, and so forth may be used. As for organic trace nutrient sources, it is desirable that the medium contains required substances such as vitamin B₁ and L-homoserine, yeast extract, and so forth in appropriate amounts. Other than the above, potassium phosphate, magnesium sulfate, iron ions, manganese ions and so forth are added in small amounts, as required. In addition, the medium used for the present invention may be either a natural medium or a synthetic medium, so long as a medium containing a carbon source, a nitrogen source, and inorganic ions, and containing other organic trace components as required is used.

Moreover, an L-amino acid which improves growth and productivity may be added. In the case of L-lysine fermentation, it is preferable to add L-threonine, L-homoserine, and/or L-isoleucine. Addition concentration is about 0.01 to 10 g/L for each.

The culture is preferably performed for 1 to 7 days under aerobic conditions. The culture temperature is preferably 24 to 37°C, and pH during the culture is preferably 5 to 9. For pH adjustment, inorganic or organic acidic or alkaline substances, ammonia gas, and so forth can be used. Collection of L-lysine from the fermentation medium can usually be attained by a combination of known methods such as ion exchange resin method and precipitation method. When L-lysine is accumulated in cells, cells can be disrupted with, for example, supersonic waves or the like, and L-lysine can be collected by the ion exchange resin method or the like from supernatant obtained by removing the cells from the cell-disrupted suspension by centrifugation.

The fermentation may also be performed by a method in which pH of the medium is controlled to be 6.5 to 9.0 during culture and to be 7.2 to 9.0 at the end of the culture, and the pressure in the fermentation tank is controlled to be positive during fermentation, or carbon dioxide or a mixed gas containing carbon dioxide is supplied to the medium so that there is a culture period where bicarbonate ions and/or carbonate ions are present in the culture medium in an amount of at least 2 g/L during the culture, and these bicarbonate ions and/or carbonate ions serve as counter ions of cations mainly consisting of L-lysine (refer to Japanese Patent Laid-open No. 2002-065287, International Patent Publication WO2006/038695).

### EXAMPLES

Hereinafter, the present invention will be still more specifically explained with reference to examples.

### Example 1: Construction of L-lysine-producing bacterium with enhanced GAPDH activity

### <1-1> Construction of plasmid for expressing gapA gene

The entire chromosomal nucleotide sequence of the *Escherichia coli* K-12 strain has already been elucidated (Science, 277, 1453-1474 (1997)). By using the synthetic oligonucleotide of SEQ ID NO: 3 as a 5' primer and the synthetic oligonucleotide of SEQ ID NO: 4, as a 3' primer, which were prepared on the basis of the nucleotide sequence of the *gapA* gene reported in the above literature, and the chromosomal DNA of *Escherichia coli* MG1655 strain as a template, PCR was performed. The purified PCR product was ligated with the vector pMW218 (Takara Bio) which had been digested with EcoRI and SacI to construct a plasmid pMW-gapA for expressing *gapA.*

### <1-2> Construction of L-lysine-producing bacterium with enhanced GAPDH activity

As an L-lysine-producing bacterium, the *Escherichia coli* WC196ΔcadAΔldc/pCABD2 (WO2006/078039) was used. This strain was deposited at National Institute of Advanced Industrial Science and Technology, International Patent Organism Depositary (Tsukuba Central 6, 1-1, Higashi 1-Chome, Tsukuba-shi, Ibaraki-ken, 305-8566, Japan) on October 7, 2008 as an international deposit and assigned an accession number of FERM BP-11027. The WC196ΔcadAΔldc/pCABD2 strain was transformed with the plasmid pMW-gapA for expressing *gapA* in a conventional manner to obtain a WC196ΔcadAΔldc/pCABD2,pMW-gapA strain.

These strains were each cultured at 37°C in an L medium containing 20 mg/L of streptomycin or 20 mg/L of streptomycin and 50 mg/L of kanamycin until final OD₆₀₀ became about 0.6, then the culture was added with an equal volume of 40% glycerol solution, and the mixture was stirred, divided into appropriate volumes, and stored at - 80°C. These mixtures will be called glycerol stocks.

### Example 2: Evaluation of L-lysine-producing ability of GAPDH activity-enhanced strain

The above glycerol stocks were thawed, and uniformly applied to an L-plate containing 20 mg/L of streptomycin, or 20 mg/L of streptomycin and 50 mg/L of kanamycin in a volume of 100 µL for each, and culture was performed at 37°C for 24 hours. The suspension of the obtained cells was diluted 101 times, OD₆₀₀ value (n) of the diluted suspension was measured, and the cell suspension in a volume corresponding to 50/n was inoculated into 20 mL of a fermentation medium containing 20 mg/L of streptomycin, or 20 mg/L of streptomycin and 50 mg/L of kanamycin in a 500-mL Sakaguchi flask, and culture was performed at 37°C for 48 hours on a reciprocally shaking culture machine (115 rpm). After the culture, amount of L-lysine accumulated in the medium was measured in a known manner (Biotec Analyzer AS210, SAKURA SEIKI).

The composition of the fermentation medium is shown below (unit: g/L).

| | |
|---|---|
| Glycerol or glucose | 40 |
| (NH₄)₂SO₄ | 24 |
| KH₂PO₄ | 1.0 |
| MgSO₄ • 7H₂O | 1.0 |
| FeSO₄ • 7H₂O | 0.01 |
| MnSO₄ • 5H₂O | 0.01 |
| Yeast Extract | 2.0 |
| | |
| CaCO₃ | 30 |

The medium was adjusted to pH 7.0 with KOH, autoclaved at 115°C for 10 minutes (provided that glycerol or glucose and MgSO₄•7H₂O were separately sterilized, and CaCO₃ was subjected to hot air sterilization at 180°C for 2 hours).

Streptomycin was added in an amount of 20 mg/L, or streptomycin and kanamycin were added in amounts of 20 mg/L and 50 mg/L, respectively.

The results are shown in Tables 1 and 2. OD means absorbance at 600 nm, Lys (g/L) means amount of L-lysine accumulated in the flask, RS (g/L) means amount of residual substrate, and Yield (%) means L-lysine yield based on the substrate.

The L-lysine-producing bacterium in which the GAPDH activity was enhanced accumulated L-lysine in a larger amount as compared to the non-enhanced strain for the both cases of using glucose and glycerol as the substrate. Furthermore, a markedly larger amount of L-lysine was accumulated when glycerol was used as the substrate as compared to the case of using glucose as the substrate.

**Table 1**

| Glucose | 24h | | | | 48h | | | |
|---|---|---|---|---|---|---|---|---|
| | OD | Lys | RS | Yield | OD | Lys | RS | Yield |
| | | (g/L) | (g/L) | (%) | | (g/L) | (g/L) | (%) |
| WC196ΔcadAΔldc /pCABD2,pMW-218 | 11.8 | 9.1 | 16.1 | 37.5 | 18.9 | 14.1 | 0.0 | 35.0 |
| WC196ΔcadAΔldc /pCABD2, pMW-gapA | 9.2 | 7.9 | 20.6 | 40.2 | 16.9 | 16.9 | 0.0 | 41.8 |

**Table 2**

| Glycerol | 24h | | | | 48h | | | |
|---|---|---|---|---|---|---|---|---|
| | OD | Lys | RS | Yield | OD | Lys | RS | Yield |
| | | (g/L) | (g/L) | (%) | | (g/L) | (g/L) | (%) |
| WCl96ΔcadAΔldc, pMW-218 | 16.8 | 10.9 | 13.3 | 36.1 | 16.3 | 15.1 | 0.0 | 34.7 |
| WC196ΔcadAΔldc, pMW-gapA | 15.0 | 14.7 | 8.4 | 42.1 | 14.6 | 18.8 | 0.0 | 43.3 |

## Claims

1. A method for producing L-lysine, comprising culturing an *Escherichia coli* which has L-lysine-producing ability and has been modified to increase glyceraldehyde 3-phosphate dehydrogenase activity in a medium to produce and accumulate L-lysine in culture, and collecting L-lysine from the culture, wherein the glyceraldehyde 3-phosphate dehydrogenase activity is increased by increasing the expression of a *gapA* gene by increasing copy number of the gene or modifying an expression control sequence of the gene, and wherein the *gapA* gene is a DNA defined in the following (a) to (d):
(a) a DNA comprising the nucleotide sequence of SEQ ID NO: 1,
(b) a DNA hybridizable with a complementary sequence of the nucleotide sequence of SEQ ID NO: 1 under stringent conditions comprising washing at salt concentrations and temperature of 0.1 X SSC, 0.1% SDS at 60°C, and encoding a protein having the glyceraldehyde 3-phosphate dehydrogenase activity,
(c) a DNA coding for a protein having the amino acid sequence of SEQ ID NO: 2, or
(d) a DNA coding for a protein having the amino acid sequence of SEQ ID NO: 2 which includes substitutions, deletions, insertions, additions or inversions of one to 20 amino acid residues, and having glyceraldehyde 3-phosphate dehydrogenase activity.

2. The method according to claim 1, wherein the medium contains glycerol as a carbon source.

## Patentansprüche

1. Verfahren zur Herstellung von L-Lysin, umfassend das Kultivieren eines Escherichia coli, welches die Fähigkeit zur Herstellung von L-Lysin hat und modifiziert wurde, um Glyceraldehyd-3-phosphat-Dehydrogenase-Aktivität in einem Medium zu steigern, um L-Lysin in Kultur herzustellen und zu akkumulieren, und das Gewinnen von L-Lysin aus der Kultur, wobei die Glyceraldehyd-3-phosphat-Dehydrogenase-Aktivität gesteigert wird durch die Steigerung der Expression eines gapA-Gens durch Steigerung der Kopienzahl des Gens oder Modifikation einer Expressionskontrollsequenz des Gens, und wobei das gapA-Gen eine DNA ist, die im folgenden in (a) bis (d) definiert ist:
(a) eine DNA, die die Nukleotidsequenz von SEQ ID Nr.: 1 umfasst,
(b) eine DNA, die mit einer komplementären Sequenz der Nukleotidsequenz von SEQ ID Nr.: 1 unter stringenten Bedingungen hybridisierbar ist, die das Waschen bei Salzkonzentrationen und Temperatur von 0,1 X SSC, 0,1 % SDS bei 60°C umfassen, und die ein Protein kodiert, das die Glyceraldehyd-3-phosphat-Dehydrogenase-Aktivität hat,
(c) eine DNA, die für ein Protein kodiert, das die Aminosäuresequenz von SEQ ID Nr.: 2 hat, oder
(d) eine DNA, die für ein Protein kodiert, das die Aminosäuresequenz von SEQ ID Nr.: 2 hat, die Substitutionen, Deletionen, Insertionen, Additionen oder Inversionen von einem bis 20 Aminosäureresten beinhaltet, und das Glyceraldehyd-3-phosphat-Dehydrogenase-Aktivität hat.

2. Verfahren gemäß Anspruch 1, wobei das Medium Glycerol als eine Kohlenstoffquelle enthält.

## Revendications

1. Procédé pour produire de la L-lysine, comprenant la culture d'un *Escherichia coli* qui a une aptitude à produire de la L-lysine et a été modifié pour augmenter l'activité glycéraldéhyde 3-phosphate déshydrogénase dans un milieu pour produire et accumuler la L-lysine dans la culture, et la collecte de la L-lysine à partir de la culture, où l'activité glycéraldéhyde 3-phosphate déshydrogénase est augmentée par augmentation de l'expression d'un gène *gapA* par augmentation du nombre de copies du gène ou modification d'une séquence de contrôle de l'expression du gène, et où le gène *gapA* est un ADN défini dans les (a) à (d) suivants :
(a) un ADN comprenant la séquence nucléotidique de SEQ ID NO : 1,
(b) un ADN hybridable avec une séquence complémentaire de la séquence nucléotidique de SEQ ID NO: 1 dans des conditions stringentes comprenant un lavage à des concentrations de sels et une température de 0,1 X SSC, 0,1 % SDS à 60°C, et codant une protéine ayant l'activité glycéraldéhyde 3-phosphate déshydrogénase,
(c) un ADN codant une protéine ayant la séquence d'aminoacides de SEQ ID NO : 2, ou
(d) un ADN codant une protéine ayant la séquence d'aminoacides de SEQ ID NO : 2 qui inclut des substitutions, des délétions, des insertions, des additions ou des inversions de un à 20 résidus d'aminoacides, et ayant une activité glycéraldéhyde 3-phosphate déshydrogénase.

2. Procédé selon la revendication 1 où le milieu contient du glycérol comme une source de carbone.
